# EUROPEAN PATENT APPLICATION

(11) **EP 2 980 091 A1**
(43) Date of publication of application: **03.02.2016**
(21) Application number: 14178989.1
(22) Date of filing: 29.07.2014
(51) Int. Cl.: C07D 498/14, A61K 31/5365, A61P 31/18

(54) **Novel hydrates of dolutegravir sodium**

(71) Applicant: LEK Pharmaceuticals d.d., 1526 Ljubljana (SI)
(72) Inventor: The designation of the inventor has not yet been filed
(74) Representative: Kluschanzoff, Harald

(57) **Abstract**

The invention relates to novel hydrates of dolutegravir sodium and to processes for their preparation. Furthermore, the invention relates to a novel crystalline form of dolutegravir sodium, which is a useful intermediate for the preparation of one of the novel hydrates. In addition, the invention relates to the use of the novel hydrates for the production of pharmaceutical compositions. Finally, the invention relates to pharmaceutical compositions comprising an effective amount of the novel hydrates and to the use of said pharmaceutical compositions in the treatment of retroviral infections such as HIV-1 infections.

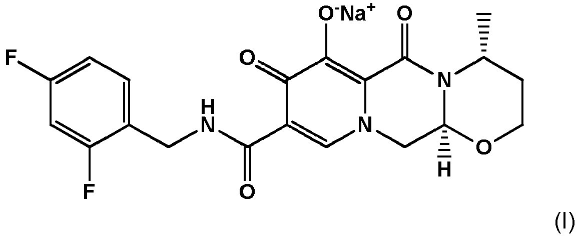

## Description

### FIELD OF THE INVENTION

The invention relates to novel hydrates of dolutegravir sodium and to processes for their preparation. Furthermore, the invention relates to a novel crystalline form of dolutegravir sodium, which is an useful intermediate for the preparation of one of the novel hydrates. In addition, the invention relates to the use of the novel hydrates for the production of pharmaceutical compositions. Finally, the invention relates to pharmaceutical compositions comprising an effective amount of the novel hydrates and to the use of said pharmaceutical compositions in the treatment of retroviral infections such as HIV-1 infections.

### BACKGROUND OF THE INVENTION

Dolutegravir, chemically designated (4*R*,12a*S*)-*N*-(2,4-difluorobenzyl)-7-hydroxy-4-methyl-6,8-dioxo-3,4,6,8,12,12a-hexahydro-2*H*-pyrido[1',2':4,5]pyrazino[2,1-*b*][1,3]oxazine-9-carboxamide, is a human immunodeficiency virus type 1 (HIV-1) integrase strand transfer inhibitor (INSTI) indicated in combination with other antiretroviral agents for the treatment of HIV-1 infection. The marketed finished dosage form (TIVICAY™) contains dolutegravir as its sodium salt, chemically denominated sodium (4*R*,12a*S*)-9-((2,4-difluorobenzyl)carbamoyl)-4-methyl-6,8-dioxo-3,4,6,8,12,12a-hexahydro-2*H*-pyrido[1',2':4,5]pyrazino[2,1-*b*][1,3]oxazin-7-olate, which is represented by the following general chemical formula (I):

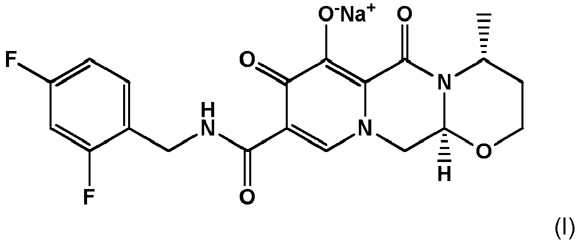

WO 2010/068253 A1 discloses a monohydrate and an anhydrous form of dolutegravir sodium as well as a crystalline form of the free compound. Processes for the preparation of said forms are also provided in the application.

WO 2013/038407 A1 discloses amorphous dolutegravir sodium and processes for preparing the same.

Hydrates of pharmaceutical drug substances are of particular interest as they provide new opportunities for preparing novel pharmaceutical compositions with improved quality, activity and/or compliance. This is due to the fact that hydrates have different physicochemical properties compared to their anhydrous counterparts such as melting point, density, habitus, chemical and physical stability, hygroscopicity, dissolution rate, solubility, bioavailability etc., which influence the formulation process and also impact the final drug product.

If an anhydrous form is selected, phase changes during the formulation process induced by hydrate formation must be avoided. This can be particularly difficult if for example wet granulation is used with a substance that is able to form hydrates like dolutegravir sodium. Hence, a stable hydrate of dolutegravir sodium would allow to easily formulate dolutegravir sodium in a controlled manner and subsequently also facilitate storage and packaging. However, the so far known dolutegravir sodium monohydrate disclosed in WO 2010/068253 A1 shows excessive water uptake when exposed to moisture and on the other hand already dehydrates below 30% relative humidity.

Therefore, there is a need for hydrates of dolutegravir sodium with improved physicochemical properties, e.g. for hydrates which are stable over a broad humidity range, in particular for hydrates absorbing only low amounts of water at elevated humidity and on the other hand preserving their crystal structure also at dry conditions. In addition, there is a need for pharmaceutical compositions comprising these hydrates.

### SUMMARY OF THE INVENTION

The present invention relates to novel hydrates of dolutegravir sodium and to processes for their preparation. Specifically, the present invention provides crystalline forms of dolutegravir sodium of formula (I) according to respective claims 1, 5 and 7, and preferred embodiment thereof according to sub-claims 2, 6 and 8, respectively. The present invention also provides processes for their preparation according to respective claims 3, 9 and 10, with preferred process embodiments being set forth in sub-claims 4 and 11, respectively. The present invention further provides the uses according to claims 12 and 15, and a pharmaceutical composition according to claim 13, while the pharmaceutical composition for therapeutic use is set forth in claim 14.

The novel hydrates are physically and chemically stable over a broad humidity range, show only low water uptakes when exposed to moisture and are even stable at dry conditions. Therefore, the novel hydrates are especially suitable for the preparation of pharmaceutical compositions.

In addition, the present invention relates to a novel crystalline form of dolutegravir sodium, which, for the first time, allows the preparation of one of the novel hydrates and is therefore a valuable intermediate.

Aspects, advantageous features and preferred embodiments of the present invention are summarized in the following items:
1) A crystalline form HxA of a compound dolutegravir sodium characterized by a powder X-ray diffractogram comprising at least five characteristic peaks at 2-Theta angles selected from 7.9 ± 0.2°, 9.4 ± 0.2°, 11.4 ± 0.2°, 11.6 ± 0.2°, 12.5 ± 0.2°, 12.8 ± 0.2°, 13.6 ± 0.2°, 15.2 ± 0.2°, 15.9 ± 0.2°, 18.4 ± 0.2°, 19.1 ± 0.2°, 19.8 ± 0.2°, 20.0 ± 0.2°, 20.8 ± 0.2°, 22.9 ± 0.2°, 23.3 ± 0.2°, 24.4 ± 0.2°, 25.2 ± 0.2°, 26.0 ± 0.2°, 27.1 ± 0.2°, 28.4 ± 0.2° and/or 29.4 ± 0.2°, preferably measured with Cu-Kα radiation, more preferably measured with Cu-Kα_{1,2} radiation having a wavelength of 0.15419 nm at room temperature.
2) A crystalline form HxA of a compound dolutegravir sodium characterized by a powder X-ray diffractogram comprising characteristic peaks at 2-Theta angles of 7.9 ± 0.2°, 9.4 ± 0.2°, 12.5 ± 0.2°, 15.9 ± 0.2° and 20.8 ± 0.2°, preferably measured with Cu-Kα radiation, more preferably measured with Cu-Kα_{1,2} radiation having a wavelength of 0.15419 nm at room temperature.
3) A crystalline form HxA of a compound dolutegravir sodium according to item 2) characterized by a powder X-ray diffractogram further comprising one or more additional characteristic peaks at 2-Theta angles of 11.4 ± 0.2°, 11.6 ± 0.2°, 13.6 ± 0.2°, 15.2 ± 0.2°, 18.4 ± 0.2°, 19.1 ± 0.2°, 19.8 ± 0.2°, 22.9 ± 0.2°, 23.3 ± 0.2°, 24.4 ± 0.2°, 26.0 ± 0.2° and/or 27.1 ± 0.2°, preferably measured with Cu-Kα radiation, more preferably measured with Cu-Kα_{1,2} radiation having a wavelength of 0.15419 nm at room temperature.
4) A crystalline form HxA of a compound dolutegravir sodium according to item 2) characterized by a powder X-ray diffractogram further comprising one or more additional characteristic peaks at 2-Theta angles of 11.6 ± 0.2°, 13.6 ± 0.2°, 15.2 ± 0.2°, 24.4 ± 0.2° and/or 26.0 ± 0.2°, preferably measured with Cu-Kα radiation, more preferably measured with Cu-Kα_{1,2} radiation having a wavelength of 0.15419 nm at room temperature.
5) A crystalline form HxA of a compound dolutegravir sodium according to item 2) characterized by a powder X-ray diffractogram comprising characteristic peaks at 2-Theta angles of 7.9 ± 0.2°, 9.4 ± 0.2°, 11.6 ± 0.2°, 12.5 ± 0.2°, 13.6 ± 0.2°, 15.2 ± 0.2°, 15.9 ± 0.2°, 20.8 ± 0.2°, 24.4 ± 0.2° and 26.0 ± 0.2°, preferably measured with Cu-Kα radiation, more preferably measured with Cu-Kα_{1,2} radiation having a wavelength of 0.15419 nm at room temperature.
6) A crystalline form HxA of a compound dolutegravir sodium according to item 2) or item 5) characterized by a powder X-ray diffractogram comprising characteristic peaks at 2-Theta angles of 7.9 ± 0.2°, 9.4 ± 0.2°, 11.4 ± 0.2°, 11.6 ± 0.2°, 12.5 ± 0.2°, 13.6 ± 0.2°, 15.2 ± 0.2°, 15.9 ± 0.2°, 18.4 ± 0.2°, 19.1 ± 0.2°, 19.8 ± 0.2°, 20.8 ± 0.2°, 22.9 ± 0.2°, 23.3 ± 0.2°, 24.4 ± 0.2°, 26.0 ± 0.2° and 27.1 ± 0.2°, preferably measured with Cu-Kα radiation, more preferably measured with Cu-Kα_{1,2} radiation having a wavelength of 0.15419 nm at room temperature.
7) A crystalline form HxA according to any of items 1 to 6 comprising 0.2 to 0.7 mol equivalents of water per mol dolutegravir sodium of formula (I) when measured at a relative humidity from 30 to 70% relative humidity and a temperature of 25 ± 0.1 °C.
8) A process for the preparation of the crystalline form HxA according to any one of items 1 to 7 comprising the steps of:
   (i) providing a suspension or a solution comprising dolutegravir in a solvent, preferably comprising at least 90 volume% methanol, more preferably about at least 95 volume% and most preferably about at least 99 volume%, wherein the suspension or the solution comprises more than 50 g, preferably at least 75 g, more preferably about 75 to 95 g, even more preferably about 75 to 85 g and most preferably about 75 to 80 g of dolutegravir per liter of solvent,
   (ii) reacting dolutegravir with sodium methoxide, preferably at a temperature of not more than 30 °C, preferably at about not more than 20 °C, more preferably at about not more than 10 °C and most preferably at about not more than 5 °C, preferably for about 1 to 24 hours, more preferably for about 1 to 12 hours and most preferably for about 1 to 2 hours, preferably under stirring, and
   (iii) optionally, increasing the reaction temperature preferably to about 10 to 30 °C, more preferably to about 15 to 30 °C and most preferably to about 20 to 25 °C and keeping the suspension or the solution at the applied temperature preferably for about 1 to 48 hours, more preferably for about 1 to 24 hours and most preferably for about 1 to 12 hours, preferably under stirring, and
   (iv) precipitating dolutegravir sodium.
   Sodium methoxide may be applied as solid or methanolic solution Solutions preferably have a sodium methoxide concentration of about 1 to 30 weight%, preferably of about 10 to 25 weight%, more preferably of about 20 to 25 weight% and most preferably of about 25 weight%. The ratio of dolutegravir and sodium methoxide employed is about 1.0 : 1.0 to 1.1, preferably a 1.0 : 1.0 ratio is used for the reaction.
9) A process according to item 8, further comprising
   (v) isolating dolutegravir sodium by separating at least a portion of the crystalline form HxA of dolutegravir sodium according to any one of the items 1 to 7 from its mother liquor, preferably by filtration or centrifugation, more preferably by filtration.
10) The process according to item8, further comprising:
   (v) isolating obtained dolutegravir sodium by filtration or centrifugation, preferably by filtration.
11) A process according to item 9 or item 10, further comprising drying the crystalline form of dolutegravir sodium obtained in step (v) at a temperature of not more than 80 °C, preferably of about not more than 50 °C, more preferably at a temperature of about not more than 40 °C, most preferably at a temperature of about 15 °C to 25 °C; and a vacuum of not more than 100 mbar, more preferably at about not more than 50 mbar and most preferably at about not more than 30 mbar.
12) A crystalline form Hy1 B of a compound dolutegravir sodium characterized by a powder X-ray diffractogram comprising at least five characteristic peaks at 2-Theta angles selected from 6.3 ± 0.2°, 7.4 ± 0.2°, 10.9 ± 0.2°, 11.1 ± 0.2°, 12.7 ± 0.2°, 16.2 ± 0.2, 18.6 ± 0.2°, 18.8 ± 0.2°, 19.7 ± 0.2°, 20.5 ± 0.2°, 21.0 ± 0.2°, 22.1 ± 0.2°, 23.0 ± 0.2°, 24.0 ± 0.2°, 24.9 ± 0.2° and/or 26.5 ± 0.2°, preferably measured with Cu-Kα radiation, more preferably measured with Cu-Kα_{1,2} radiation having a wavelength of 0.15419 nm at room temperature.
13) A crystalline form Hy1 B of a compound dolutegravir sodium characterized by a powder X-ray diffractogram comprising characteristic peaks at 2-Theta angles of 6.3 ± 0.2°, 7.4 ± 0.2°, 11.1 ± 0.2°, 12.7 ± 0.2° and 16.2 ± 0.2°, preferably measured with Cu-Kα radiation, more preferably measured with Cu-Kα_{1,2} radiation having a wavelength of 0.15419 nm at room temperature.
14) A crystalline form Hy1B of a compound dolutegravir sodium according to item 13) characterized by a powder X-ray diffractogram further comprising one or more additional characteristic peaks at 2-Theta angles of 10.9 ± 0.2°, 18.6 ± 0.2°, 18.8 ± 0.2°, 19.7 ± 0.2°, 20.5 ± 0.2°, 21.0 ± 0.2°, 22.1 ± 0.2°, 23.0 ± 0.2°, 24.0 ± 0.2°, 24.9 ± 0.2° and/or 26.5 ± 0.2°, preferably measured with Cu-Kα radiation, more preferably measured with Cu-Kα_{1,2} radiation having a wavelength of 0.15419 nm at room temperature.
15) A crystalline form Hy1B of a compound dolutegravir sodium according to item 13) characterized by a powder X-ray diffractogram further comprising one or more additional characteristic peaks at 2-Theta angles of 10.9 ± 0.2°, 18.6 ± 0.2°, 18.8 ± 0.2°, 20.5 ± 0.2°, 21.0 ± 0.2° and/or 23.0 ± 0.2°, preferably measured with Cu-Kα radiation, more preferably measured with Cu-Kα_{1,2} radiation having a wavelength of 0.15419 nm at room temperature.
16) A crystalline form Hy1B of a compound dolutegravir sodium according to item 13) characterized by a powder X-ray diffractogram comprising characteristic peaks at 2-Theta angles of 6.3 ± 0.2°, 7.4 ± 0.2°, 10.9 ± 0.2°, 11.1 ± 0.2°, 12.7 ± 0.2°, 16.2 ± 0.2°, 18.6 ± 0.2°, 18.8 ± 0.2°, 20.5 ± 0.2°, 21.0 ± 0.2° and 23.0 ± 0.2°, preferably measured with Cu-Kα radiation, more preferably measured with Cu-Kα_{1,2} radiation having a wavelength of 0.15419 nm at room temperature.
17) A crystalline form Hy1 B of a compound dolutegravir sodium according to item 13) or item 16) characterized by a powder X-ray diffractogram comprising characteristic peaks at 2-Theta angles of 6.3 ± 0.2°, 7.4 ± 0.2°, 10.9 ± 0.2°, 11.1 ± 0.2°, 12.7 ± 0.2°, 16.2 ± 0.2°, 18.6 ± 0.2°, 18.8 ± 0.2°, 19.7 ± 0.2°, 20.5 ± 0.2°, 21.0 ± 0.2°, 22.1 ± 0.2°, 23.0 ± 0.2°, 24.0 ± 0.2°, 24.9 ± 0.2° and 26.5 ± 0.2°, preferably measured with Cu-Kα radiation, more preferably measured with Cu-Kα_{1,2} radiation having a wavelength of 0.15419 nm at room temperature.
18) A crystalline form Hy1 B according to any of items 12 to 17 comprising 0.7 to 1.3 mol equivalents of water per mol dolutegravir sodium when measured at a relative humidity from 30 to 90% and a temperature of 25 ± 0.1 °C.
19) A process for the preparation of the crystalline form Hy1 B according to any one of the items 12 to 18 comprising subjecting the crystalline form S_{EtOH/H2O} according to any one of items 20 to 26 to an atmosphere having a relative humidity of at least 75 ± 5% and a temperature of at least 40 °C, preferably for about 12 to 168 hours, more preferably for about 12 to 72 hours and most preferably for about 12 to 24 hours.
20) A crystalline form S_{EtOH/H2O} of a compound dolutegravir sodium characterized by a powder X-ray diffractogram comprising at least five characteristic peaks at 2-Theta angles selected from 6.5 ± 0.2°, 7.0 ± 0.2°, 10.6 ± 0.2°, 11.0 ± 0.2°, 13.3 ± 0.2°, 16.0 ± 0.2°, 18.0 ± 0.2°, 19.1 ± 0.2°, 20.3 ± 0.2°, 20.8 ± 0.2° and/or 22.6 ± 0.2°, preferably measured with Cu-Kα radiation, more preferably measured with Cu-Kα_{1,2} radiation having a wavelength of 0.15419 nm at room temperature.
21) A crystalline form S_{EtOH/H2O} of a compound dolutegravir sodium characterized by a powder X-ray diffractogram comprising characteristic peaks at 2-Theta angles of 6.5 ± 0.2°, 7.0 ± 0.2°, 13.3 ± 0.2°, 16.0 ± 0.2° and 22.6 ± 0.2°, preferably measured with Cu-Kα radiation, more preferably measured with Cu-Kα_{1,2} radiation having a wavelength of 0.15419 nm at room temperature.
22) A crystalline form S_{EtOH/H2O} of a compound dolutegravir sodium according to item 21) characterized by a powder X-ray diffractogram further comprising one or more additional characteristic peaks at 2-Theta angles of 10.6 ± 0.2°, 11.0 ± 0.2°, 18.0 ± 0.2°, 19.1 ± 0.2°, 20.3 ± 0.2° and/or 20.8 ± 0.2°, preferably measured with Cu-Kα radiation, more preferably measured with Cu-Kα_{1,2} radiation having a wavelength of 0.15419 nm at room temperature.
23) A crystalline form S_{EtOH/H2O} of a compound dolutegravir sodium according to item 21) characterized by a powder X-ray diffractogram further comprising one or more additional characteristic peaks at 2-Theta angles of 10.6 ± 0.2°, 11.0 ± 0.2°, 18.0 ± 0.2°, 19.1 ± 0.2° and/or 20.3 ± 0.2°, preferably measured with Cu-Kα radiation, more preferably measured with Cu-Kα_{1,2} radiation having a wavelength of 0.15419 nm at room temperature.
24) A crystalline form S_{EtOH/H2O} of a compound dolutegravir sodium according to item 21) characterized by a powder X-ray diffractogram comprising characteristic peaks at 2-Theta angles of 6.5 ± 0.2°, 7.0 ± 0.2°, 10.6 ± 0.2°, 11.0 ± 0.2°, 13.3 ± 0.2°, 16.0 ± 0.2°, 18.0 ± 0.2°, 19.1 ± 0.2°, 20.3 ± 0.2° and 22.6 ± 0.2°, preferably measured with Cu-Kα radiation, more preferably measured with Cu-Kα_{1,2} radiation having a wavelength of 0.15419 nm at room temperature.
25) A crystalline form S_{EtOH/H2O} of a compound dolutegravir sodium according to item 21) or item 24 characterized by a powder X-ray diffractogram comprising characteristic peaks at 2-Theta angles of 6.5 ± 0.2°, 7.0 ± 0.2°, 10.6 ± 0.2°, 11.0 ± 0.2°, 13.3 ± 0.2°, 16.0 ± 0.2°, 18.0 ± 0.2°, 19.1 ± 0.2°, 20.3 ± 0.2°, 20.8 ± 0.2° and 22.6 ± 0.2°, preferably measured with Cu-Kα radiation, more preferably measured with Cu-Kα_{1,2} radiation having a wavelength of 0.15419 nm at room temperature.
26) The crystalline form S_{EtOH/H2O} according to any of items 20 to 25 comprising 0.3 mol equivalents of ethanol and 0.2 mol equivalents of water per mol dolutegravir sodium when measured at a relative humidity of 25% and a temperature from 15 to 25 °C.
27) A process for the preparation of the crystalline form S_{EtOH/H2O} according to any one of items 20 to 26 comprising the steps of:
   (i) providing a suspension or a solution of dolutegravir in a solvent comprising at least 90 volume% ethanol, preferably about at least 96 volume% ethanol and more preferably about at least 99 volume% ethanol, preferably comprising about 10 to 100 g, more preferably about 10 to 50 g and most preferably about 10 to 25 g of dolutegravir per liter solvent
   (ii) reacting dolutegravir with sodium ethoxide at a temperature of not more than 45 °C, preferably at a temperature of not more than 40 °C, preferably at about not more than 35 °C, and most preferably at about not more than 30 °C, preferably for about 1 to 24 hours, more preferably for about 1 to 12 hours, even more preferably for about 1 to 6 hours and most preferably for about 1 to 2 hours, preferably under stirring, and
   (iii) precipitating dolutegravir sodium.
   Sodium methoxide may be applied as solid or methanolic solution Solutions preferably have a sodium methoxide concentration of about 1 to 25 weight%, preferably of about 10 to 21 weight%, and most preferably of about 21 weight%. The ratio of dolutegravir and sodium ethoxide employed is about 1.0: 0.8 to 2.0, preferably about 1.0 : 0.9 to 1.5, more preferably about 1.0 : 0.9 to 1.2 and most preferably a 1.0 : 1.0 ratio is used.
28) The process according to item 27, further comprising
   (iv) isolating dolutegravir sodium by separating at least a portion of the crystalline form S_{EtOH/H2O} of dolutegravir sodium according to any one of the items 20 to 26 from its mother liquor, preferably by filtration or centrifugation, more preferably by filtration.
29) The process according to item 27, further comprising:
   (iv) isolating obtained dolutegravir sodium by filtration or centrifugation, preferably by filtration.
30) The process according to item 28 or item 29, further comprising drying the crystalline form S_{EtOH/H2O} of dolutegravir sodium obtained in step (iv) at a temperature of not more than 80 °C, preferably of about not more than 50 °C, more preferably at a temperature of about not more than 40 °C, most preferably at a temperature of about 15 °C to 25 °C; and a vacuum of not more than 100 mbar, more preferably at about not more than 50 mbar and most preferably at about not more than 30 mbar.
31) Use of the crystalline form HxA according to any one of items 1 to 7 or the crystalline form Hy1 B according to any one of items 12 to 18 or mixtures thereof for the preparation of a pharmaceutical composition.
32) Use of the crystalline forms obtainable or obtained by a process according to any one of items 8 to 11 or item 19 or mixtures thereof for the preparation of a pharmaceutical composition.
33) A pharmaceutical composition comprising an effective amount of the crystalline form HxA according to any one of items 1 to 7 or the crystalline form Hy1 B according to any one of items 12 to 18 or mixtures thereof and at least one pharmaceutically acceptable excipient.
34) A pharmaceutical composition comprising an effective amount of the crystalline forms obtainable or obtained by a process according to any one of items 8 to 11 or item 19 or mixtures thereof and at least one pharmaceutically acceptable excipient.
35) The pharmaceutical composition according to item 33 or item 34 for use in a method for the treatment treating of human immunodefiency virus type 1 (HIV-1) infection in human.
30) Use of the crystalline form according to any one of items 20 to 26 for the preparation of the crystalline form Hy1 B according to any one of items 12 to 18.
31) Use of the crystalline form obtainable or obtained by a process according to any one of items 27 to 30 for the preparation of the crystalline form Hy1 B according to any one of items 12 to 18.

In the context of the present invention the following abbreviations have the indicated meaning, unless explicitly stated otherwise:
- PXRD: powder X-ray diffraction/ diffractogram
- TGA: thermogravimetric analyses
- DSC: differential scanning calorimetry
- GMSD: gravimetric moisture sorption/ desorption
- ¹H-NMR: proton nuclear magnetic resonance
- RT: room temperature
- RH: relative humidity
- m: mass
- V: volume
- MH: monohydrate
- MeOH: methanol
- conc.: concentration

### BRIEF DESCRIPTION OF THE FIGURES

- Figure 1:: Representative PXRD of dolutegravir sodium form HxA
- Figure 2:: Representative DSC curve of dolutegravir sodium form HxA equilibrated at about 30% RH and RT
- Figure 3:: Representative TGA curve of dolutegravir sodium form HxA equilibrated at about 30% RH and RT
- Figure 4:: Representative PXRD of dolutegravir sodium form Hy1 B
- Figure 5:: Representative DSC curve of dolutegravir sodium form Hy1 B equilibrated at about 30% RH and RT
- Figure 6:: Representative TGA curve of dolutegravir sodium form Hy1 B equilibrated at about 30% RH and RT
- Figure 7:: Representative PXRD of dolutegravir sodium form S_{EtOH/H2O}
- Figure 8:: Representative DSC curve of dolutegravir sodium form S_{EtOH/H2O} equilibrated at about 25% RH and RT
- Figure 9:: Representative TGA curve of dolutegravir sodium form S_{EtOH/H2O} equilibrated at about 25% RH and RT
- Figure 10:: Gravimetric moisture sorption/ desorption isotherms of dolutegravir sodium form HxA
- Figure 11:: Gravimetric moisture sorption/ desorption isotherms of dolutegravir sodium form Hy1 B
- Figure 12:: Gravimetric moisture sorption/ desorption isotherms of dolutegravir sodium monohydrate of WO 2010/068253 A1

### DETAILED DESCRIPTION OF THE INVENTION

The invention is described below in further detail by embodiments, without being limited thereto.

As used herein the term "room temperature" indicates that the applied temperature is not critical and that no exact temperature value has to be kept. Usually, "room temperature" is understood to mean temperatures between 15 and 25 °C [see e.g. European Pharmacopoeia 8.2, 1.2 (2014)].

The term "peaks" used herein corresponds to its typical meaning in the art of XRPD. It may generally mean peaks clearly visible in XRPD patterns. Further, a "peak" may, in a corresponding XRPD pattern, resemble a single peak at a given specified position, or it may represent a double-peak or multiple peaks around a given specified (central) position of the denoted "peak"

The term "essentially the same" with reference to PXRD means that variabilities in peak positions and relative intensities of the peaks are to be taken into account. For example, a typical precision of the 2-Theta values is in the range of ± 0.2° 2-Theta. Thus, the diffraction peak of form HxA that usually appears at 9.4° 2-Theta for example can appear between 9.2° and 9.6° 2-Theta on most X-ray diffractometers under standard conditions. Furthermore, one skilled in the art will appreciate that relative peak intensities will show inter-apparatus variability as well as variability due to degree of crystallinity, preferred orientation, sample preparation and other factors known to those skilled in the art and should be taken as qualitative measure only.

In an aspect, the present invention relates to a novel hydrate of dolutegravir sodium, above and hereinafter also designated form HxA.

Form HxA is a monosodium salt of dolutegravir. Its molar ratio of dolutegravir and sodium typically and preferably lies in a range of about 1.0 : 0.9 to 1.1, more preferably of about 1.0 : 1.0.

Form HxA can be characterized by a PXRD comprising at least five peaks at 2-Theta angles selected from 7.9 ± 0.2°, 9.4 ± 0.2°, 11.4 ± 0.2°, 11.6 ± 0.2°, 12.5 ± 0.2°, 12.8 ± 0.2°, 13.6 ± 0.2°, 15.2 ± 0.2°, 15.9 ± 0.2°, 18.4 ± 0.2°, 19.1 ± 0.2°, 19.8 ± 0.2°, 20.0 ± 0.2°, 20.8 ± 0.2°, 22.9 ± 0.2°, 23.3 ± 0.2°, 24.4 ± 0.2°, 25.2 ± 0.2°, 26.0 ± 0.2°, 27.1 ± 0.2°, 28.4 ± 0.2° and/or 29.4 ± 0.2°.

Preferably, form HxA is characterized by a PXRD comprising characteristic peaks at 2-Theta angles of 7.9 ± 0.2°, 9.4 ± 0.2°, 12.5 ± 0.2°, 15.9 ± 0.2° and 20.8 ± 0.2°. Form HxA may be further characterized by a PXRD further comprising one or more additional characteristic peaks at 2-Theta angles of 11.4 ± 0.2°, 11.6 ± 0.2°, 13.6 ± 0.2°, 15.2 ± 0.2°, 18.4 ± 0.2°, 19.1 ± 0.2°, 19.8 ± 0.2°, 22.9 ± 0.2°, 23.3 ± 0.2°, 24.4 ± 0.2°, 26.0 ± 0.2° and/or 27.1 ± 0.2°. Preferably, form HxA may be further characterized by a PXRD further comprising one or more additional characteristic peaks at 2-Theta angles of 11.6 ± 0.2°, 13.6 ± 0.2°, 15.2 ± 0.2°, 24.4 ± 0.2° and/or 26.0 ± 0.2°.

More preferably, form HxA is characterized by a PXRD comprising characteristic at 2-Theta angles of 7.9 ± 0.2°, 9.4 ± 0.2°, 11.6 ± 0.2°, 12.5 ± 0.2°, 13.6 ± 0.2°, 15.2 ± 0.2°, 15.9 ± 0.2°, 20.8 ± 0.2°, 24.4 ± 0.2° and 26.0 ± 0.2°.

Even more preferably, form HxA is characterized by a PXRD comprising characteristic at 2-Theta angles of 7.9 ± 0.2°, 9.4 ± 0.2°, 11.4 ± 0.2°, 11.6 ± 0.2°, 12.5 ± 0.2°, 13.6 ± 0.2°, 15.2 ± 0.2°, 15.9 ± 0.2°, 18.4 ± 0.2°, 19.1 ± 0.2°, 19.8 ± 0.2°, 20.8 ± 0.2°, 22.9 ± 0.2°, 23.3 ± 0.2°, 24.4 ± 0.2°, 26.0 ± 0.2° and 27.1 ± 0.2°.

Even more preferably, form HxA is characterized by a PXRD comprising characteristic at 2-Theta angles of 7.9 ± 0.2°, 9.4 ± 0.2°, 11.4 ± 0.2°, 11.6 ± 0.2°, 12.5 ± 0.2°, 12.8 ± 0.2°, 13.6 ± 0.2°, 15.2 ± 0.2°, 15.9 ± 0.2°, 18.4 ± 0.2°, 19.1 ± 0.2°, 19.8 ± 0.2°, 20.0 ± 0.2°, 20.8 ± 0.2°, 22.9 ± 0.2°, 23.3 ± 0.2°, 24.4 ± 0.2°, 25.2 ± 0.2°, 26.0 ± 0.2°, 27.1 ± 0.2°, 28.4 ± 0.2° and 29.4 ± 0.2°.

XPRDs of form HxA according to the above aspect are measured preferably with Cu-Kα radiation, more preferably with Cu-Kα_{1,2} radiation having a wavelength of 0.15419 nm at room temperature.

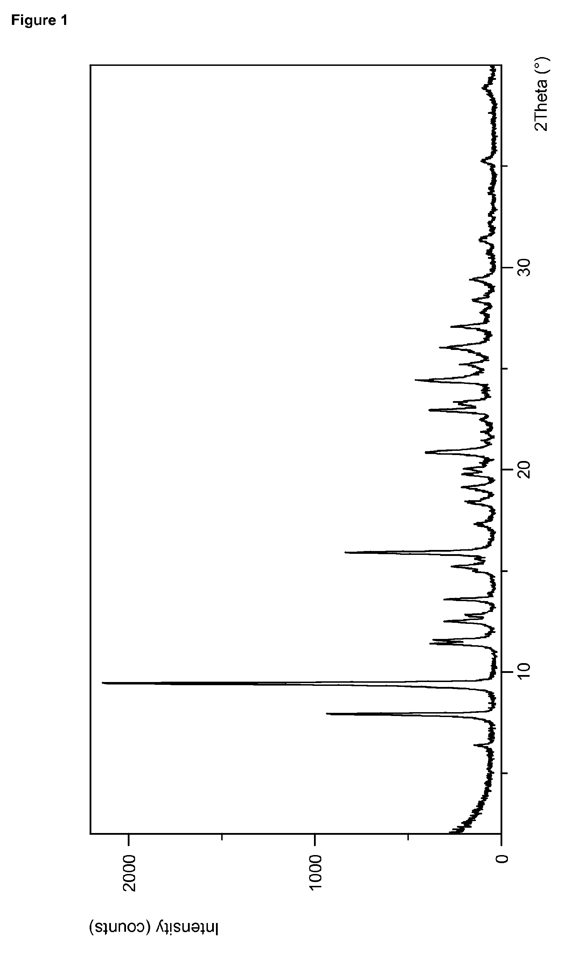

A representative PXRD of form HxA is displayed in figure 1 herein. Additionally, form HxA can be characterized by showing a PXRD essentially the same as displayed in figure 1 when measured at room temperature with Cu-Kα_{1,2} radiation having a wavelength of 0.15419 nm.

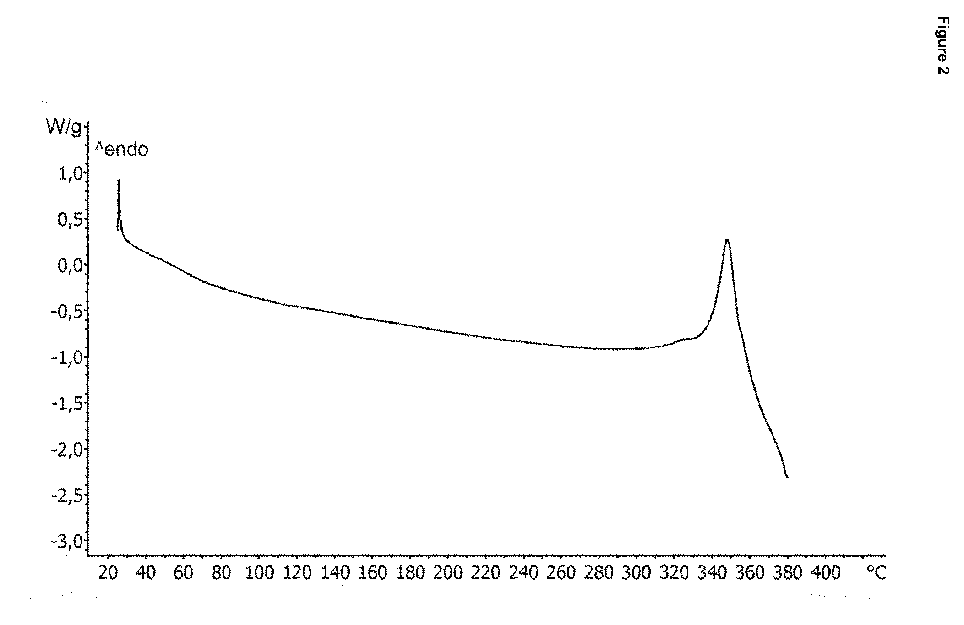

Furthermore, a representative DSC curve of form HxA equilibrated at about 30% RH and RT is displayed in figure 2 herein. The DSC curve of form HxA shows an endotherm at about 330 to 355 °C with an onset temperature of about 338 °C and a peak maximum at about 348 °C which is due to melting and decomposition respectively.

Therefore, alternatively or additionally, form HxA can be characterized by a DSC curve comprising an endotherm at 330 to 355 °C when measured at a heating rate of 10 °C/min.

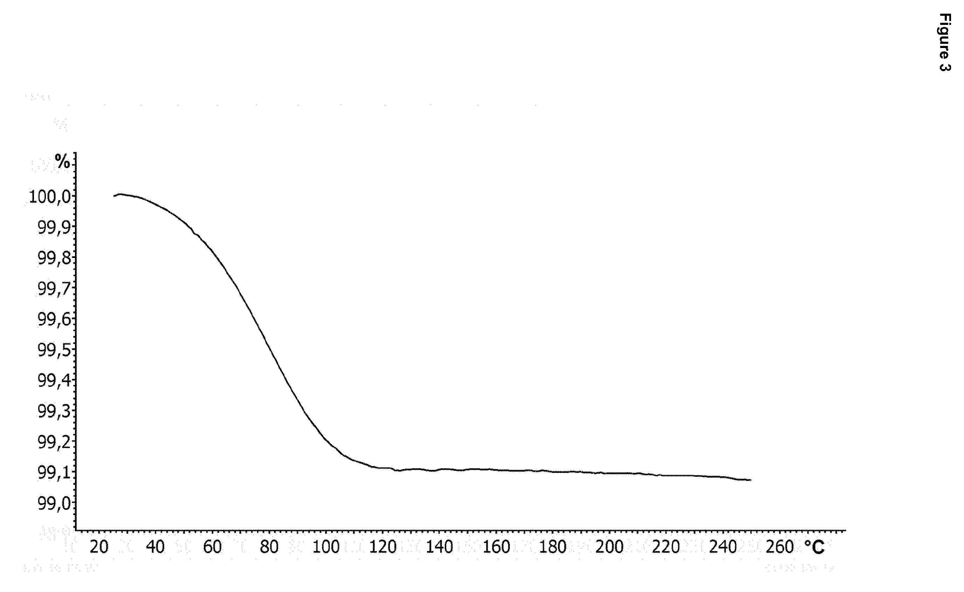

In addition, a representative TGA curve of form HxA equilibrated at about 30% RH and RT is provided in figure 3 herein. The TGA curve of form HxA shows a weight loss of about 0.9% from 25 to 120 °C. Coulometric Karl-Fischer titration of form HxA equilibrated at about 30% RH and RT revealed a water content of about 0.9% corresponding to 0.2 mol of water per mol of dolutegravir sodium. No organic solvent, in particular no methanol was traceable by ¹H-NMR.

Hence, alternatively or additionally, form HxA can be characterized by comprising not more than 0.5%, preferably not more than 0.4%, more preferably not more than 0.3% and most preferably not more than 0.2%, for example not more than 0.1% organic solvent. Alternatively or additionally, form HxA can be characterized by comprising not more than 0.3%, preferably not more than 0.2%, more preferably not more than 0.1% and most preferably not more than 0.05% methanol.

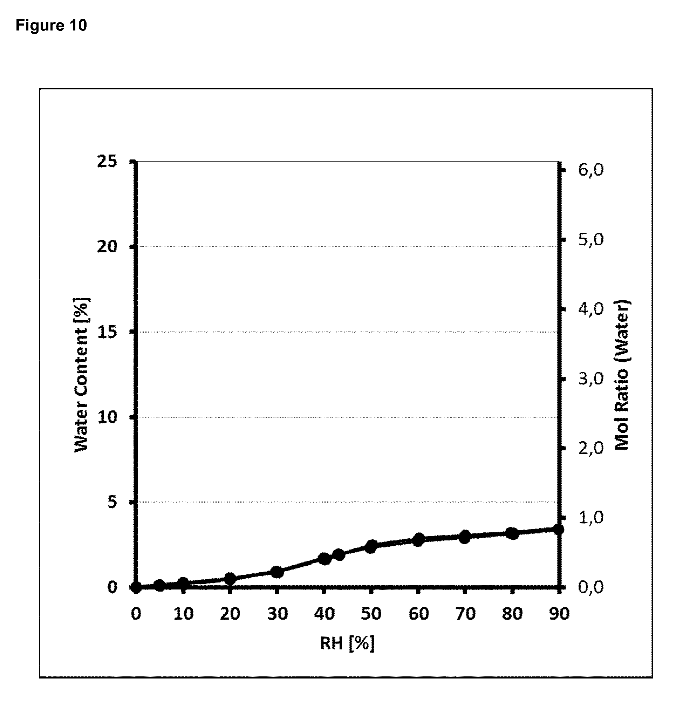

Representative gravimetric moisture sorption/desorption isotherms of form HxA are displayed in figure 10 herein. Form HxA, for example, shows a water content of about 0.9% corresponding to 0.2 mol of water per mol of dolutegravir sodium at about 30% RH and a water content of about 2.9 to 3.0% corresponding to 0.7 mol of water per mol of dolutegravir sodium at about 70% RH measured at temperature of 25 ± 0.1 °C. At 95% RH and 25 ± 0.1 °C the sample shows a maximum water content of about 3.8%, which corresponds to 0.9 mol of water per mol of dolutegravir sodium. From the GMSD isotherms displayed in figure 10 herein it becomes clear that water uptake and loss respectively are reversible and that the water content of form HxA depends on the relative humidity of the surrounding atmosphere, which is typical for non-stoichiometric hydrates.

Therefore, alternatively or additionally, form HxA can be characterized by comprising 0.2 to 0.7 mol of water per mol of dolutegravir sodium when measured at temperature of 30 to 70% RH and 25 ± 0.1 °C.

Furthermore, in a further aspect the present invention relates to a process for the preparation of form HxA of dolutegravir sodium comprising the steps of:
(i) providing a suspension or a solution of dolutegravir in a solvent comprising methanol, wherein the suspension or the solution comprises more than 50 g, preferably at least 75 g dolutegravir per liter solvent,
(ii) reacting dolutegravir with sodium methoxide at a temperature of about not more than 30 °C and
(iii) precipitating dolutegravir sodium.

Dolutegravir, which is used as starting material for form HxA production can for example be prepared in accordance with the procedures disclosed in examples 1 a to 1k or 3a to 3k of WO 2010/068253 A1.

In a first step, a suspension or a solution, preferably a suspension, of dolutegravir in a solvent comprising methanol is prepared. Preferably, the methanol concentration of the solvent used for preparing the mixture is about at least 90 volume%, more preferably about at least 95 volume% and most preferably about at least 99 volume%. Moreover, the water content of the solvent preferably is about not more than 10 volume%, more preferably about not more than 5 volume% and most preferably about not more than 1 volume%.

Essentially, the initial suspension or solution comprises more than 50 g, preferably about at least 75 g dolutegravir per liter solvent, for example about 75 to 100 g, more preferably about 75 to 95 g, even more preferably about 75 to 85 g and most preferably about 75 to 80 g dolutegravir per liter solvent may be applied.

In a next step dolutegravir is reacted with sodium methoxide by adding sodium methoxide to the suspension or the solution or *vice versa.* Sodium methoxide may be applied as solid or methanolic solution, whereas solutions which may be applied in the present process preferably have a sodium methoxide concentration of about 1 to 30 weight%, preferably of about 10 to 25 weight%, more preferably of about 20 to 25 weight% and most preferably of about 25 weight%. The ratio of dolutegravir and sodium methoxide employed is about 1.0 : 1.0 to 1.1, preferably a 1.0 : 1.0 ratio is used for the reaction.

Essentially, when combining sodium methoxide and dolutegravir, the reaction temperature is kept at about not more than 30 °C, preferably at about not more than 20 °C, more preferably at about not more than 10 °C and most preferably at about not more than 5 °C. The suspension or the solution is preferably kept at the applied temperature for about 1 to 24 hours, more preferably for about 1 to 12 hours and most preferably for about 1 to 2 hours, preferably under stirring. Thereafter, the reaction temperature is increased preferably to about 10 to 30 °C, more preferably to about 15 to 30 °C and most preferably to about 20 to 25 °C. The suspension or the solution is preferably kept at the applied temperature for about 1 to 48 hours, more preferably for about 1 to 24 hours and most preferably for about 1 to 12 hours, preferably under stirring.

Subsequently, at least a portion of the obtained dolutegravir sodium crystals may be collected by any conventional method such as filtration or centrifugation, preferably by filtration.

Finally, the isolated dolutegravir sodium crystals may be dried at a temperature of about not more than 80 °C, preferably of about not more than 60 °C, more preferably of about not more than 50 °C and most preferably the crystals are dried at a temperature of about not more than 40 °C for example at about room temperature. Drying may be performed for about 1 to 72 hours, preferably for about 2 to 48 hours, more preferably for about 4 to 24 hours and most preferably for about 6 to 18 hours. Drying may be performed under vacuum preferably at about not more than 100 mbar, more preferably at about not more than 50 mbar and most preferably at about not more than 30 mbar, for example at about 20 to 30 mbar.

Moreover, in another aspect the present invention relates to a novel hydrate of dolutegravir sodium, above and hereinafter also designated form Hy1 B.

Form Hy1 B is a monosodium salt of dolutegravir. Its molar ratio of dolutegravir and sodium typically and preferably lies in a range of about 1.0 : 0.9 to 1.1, more preferably of about 1.0 : 1.0.

Form Hy1 B can be characterized by a PXRD comprising at least five peaks at 2-Theta angles selected from 6.3 ± 0.2°, 7.4 ± 0.2°, 10.9 ± 0.2°, 11.1 ± 0.2°, 12.7 ± 0.2°, 16.2 ± 0.2, 18.6 ± 0.2°, 18.8 ± 0.2°, 19.7 ± 0.2°, 20.5 ± 0.2°, 21.0 ± 0.2°, 22.1 ± 0.2°, 23.0 ± 0.2°, 24.0 ± 0.2°, 24.9 ± 0.2° and/or 26.5 ± 0.2°.

Preferably, form Hy1 B is characterized by a PXRD comprising characteristic peaks at 2-Theta angles of 6.3 ± 0.2°, 7.4 ± 0.2°, 11.1 ± 0.2°, 12.7 ± 0.2° and 16.2 ± 0.2°. Form Hy1B may be further characterized by a PXRD further comprising one or more additional characteristic peaks at 2-Theta angles of 10.9 ± 0.2°, 18.6 ± 0.2°, 18.8 ± 0.2°, 19.7 ± 0.2°, 20.5 ± 0.2°, 21.0 ± 0.2°, 22.1 ± 0.2°, 23.0 ± 0.2°, 24.0 ± 0.2°, 24.9 ± 0.2° and/or 26.5 ± 0.2°. Preferably, form Hy1B may be further characterized by a PXRD further comprising one or more additional characteristic peaks at 2-Theta angles of 10.9 ± 0.2°, 18.6 ± 0.2°, 18.8 ± 0.2°, 20.5 ± 0.2°, 21.0 ± 0.2° and/or 23.0 ± 0.2°.

More preferably, form Hy1 B is characterized by a PXRD comprising characteristic peaks at 2-Theta angles of 6.3 ± 0.2°, 7.4 ± 0.2°, 10.9 ± 0.2°, 11.1 ± 0.2°, 12.7 ± 0.2°, 16.2 ± 0.2°, 18.6 ± 0.2°, 18.8 ± 0.2°, 20.5 ± 0.2°, 21.0 ± 0.2° and 23.0 ± 0.2°

Even more preferably, form Hy1 B of a compound dolutegravir sodium is characterized by a powder X-ray diffractogram comprising characteristic peaks at 2-Theta angles of 6.3 ± 0.2°, 7.4 ± 0.2°, 10.9 ± 0.2°, 11.1 ± 0.2°, 12.7 ± 0.2°, 16.2 ± 0.2°, 18.6 ± 0.2°, 18.8 ± 0.2°, 19.7 ± 0.2°, 20.5 ± 0.2°, 21.0 ± 0.2°, 22.1 ± 0.2°, 23.0 ± 0.2°, 24.0 ± 0.2°, 24.9 ± 0.2° and 26.5 ± 0.2°.

XPRDs of form Hy1B according to the above aspect are measured preferably with Cu-Kα radiation, more preferably with Cu-Kα_{1,2} radiation having a wavelength of 0.15419 nm at room temperature.

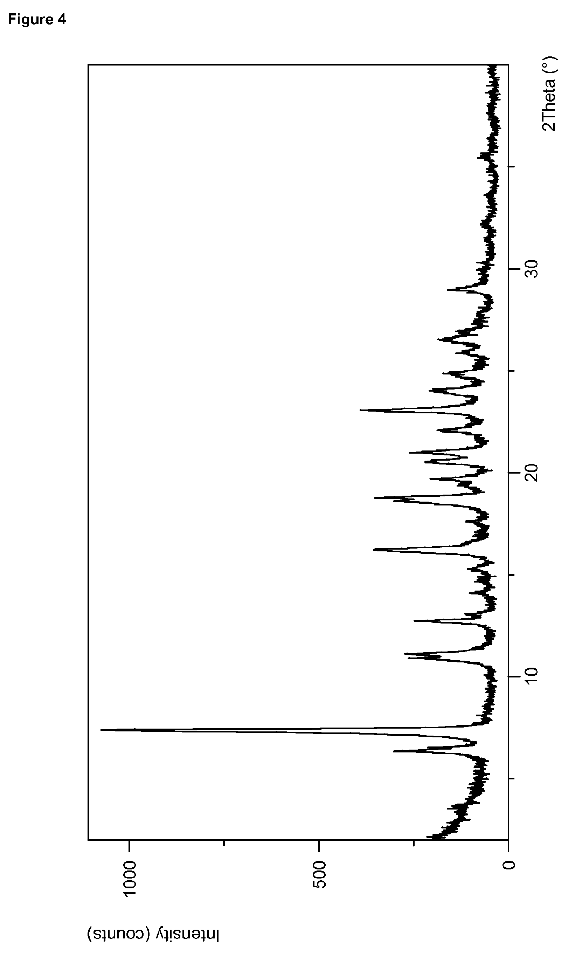

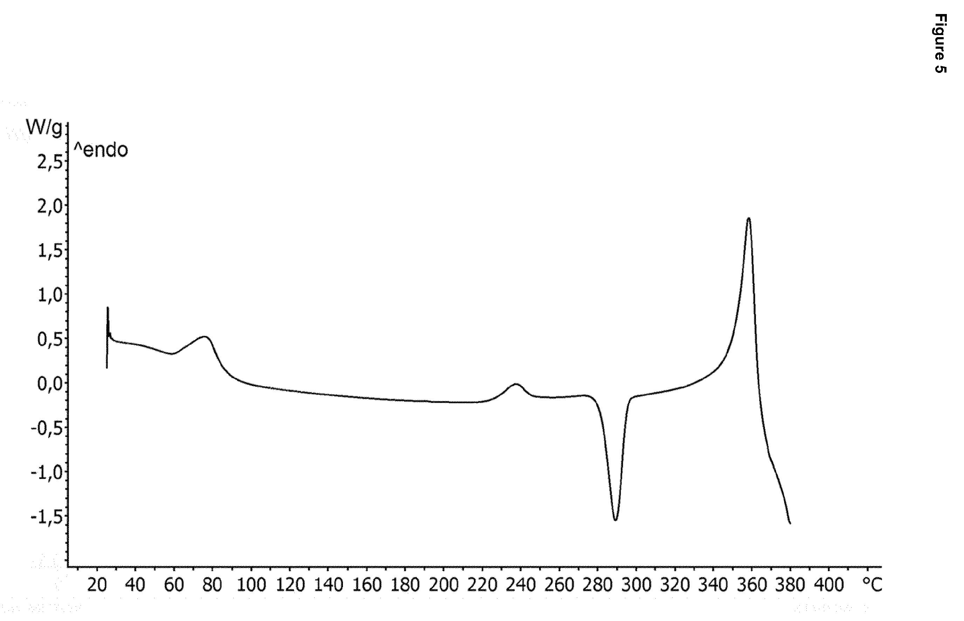

A representative PXRD of form Hy1 B is displayed in figure 4 herein. Additionally, form Hy1 B can be characterized by showing a PXRD essentially the same as displayed in figure 4 when measured at room temperature with Cu-Kα_{1,2} radiation having a wavelength of 0.15419 nm. Furthermore, a representative DSC curve of form Hy1 B equilibrated at about 30% RH and RT is displayed in figure 5 herein. The DSC curve of form Hy1 B shows a first endotherm at about 60 to 90 °C with a peak maximum at about 77 °C, which is due to release of water. The second endotherm at about 220 to 250 °C with a peak maximum at about 237 °C is caused by melting. The exotherm, which occurs at about 275 to 300 °C and has a peak maximum at about 289 °C indicates that the anhydrous form of WO 2010/068253 A1 crystallizes from the melt. The final endotherm at about 340 to 370 °C with an onset temperature of about 350 °C and a peak maximum at about 358 °C is due to melting and decomposition respectively of the obtained anhydrous form of WO 2010/068253 A1.

Therefore, alternatively or additionally, form Hy1B can be characterized by a DSC curve comprising an endotherm at about 60 to 90 °C and/or an endotherm at about 220 to 250 °C when measured at a heating rate of 10 °C/min. Form Hy1 B can be further characterized by a DSC curve comprising an additional exotherm at about 275 to 300 °C when measured at a heating rate of 10 °C/min.

Alternatively or additionally, form Hy1 B can be characterized by melting in the range of about 220 to 250 °C when measured with DSC at a heating rate of 10 °C/min.

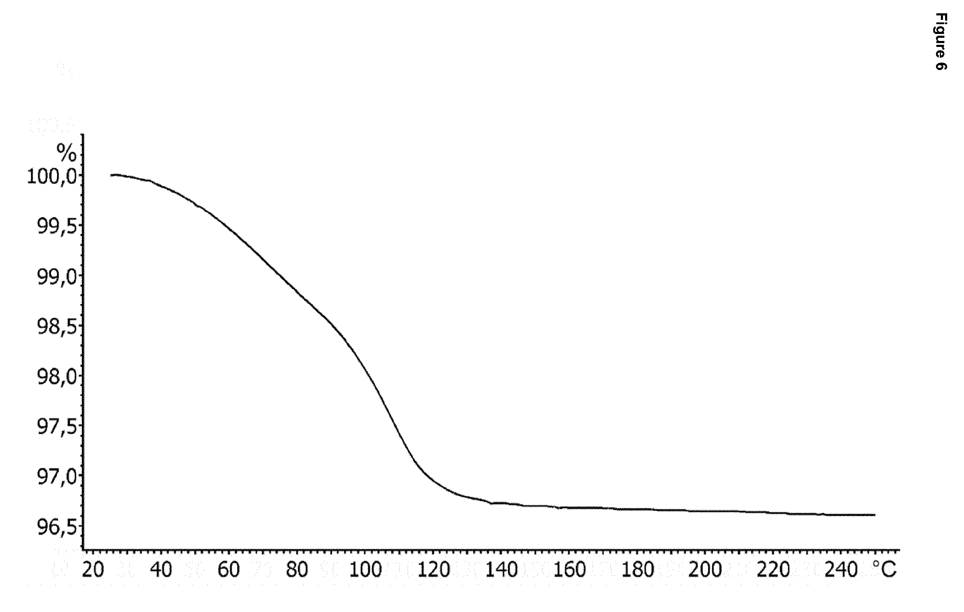

In addition, a representative TGA curve of form Hy1 B equilibrated at about 30% RH and RT is provided in figure 6 herein. The TGA curve of form Hy1 B shows a weight loss of about 3.3% from 25 to 150 °C. Coulometric Karl-Fischer titration of form Hy1 B equilibrated at about 30% RH and RT revealed a water content of about 3.3% corresponding to 0.8 mol of water per mol of dolutegravir sodium. No organic solvent, in particular no ethanol was traceable by ¹H-NMR.

Hence, alternatively or additionally, form Hy1 B can be characterized by comprising not more than 0.5%, preferably not more than 0.4%, more preferably not more than 0.3% and most preferably not more than 0.2%, for example not more than 0.1% organic solvent. Alternatively or additionally, form Hy1B can be characterized by comprising not more than 0.5%, preferably not more than 0.4%, more preferably not more than 0.3% and most preferably not more than 0.2%, for example not more than 0.1 % ethanol.

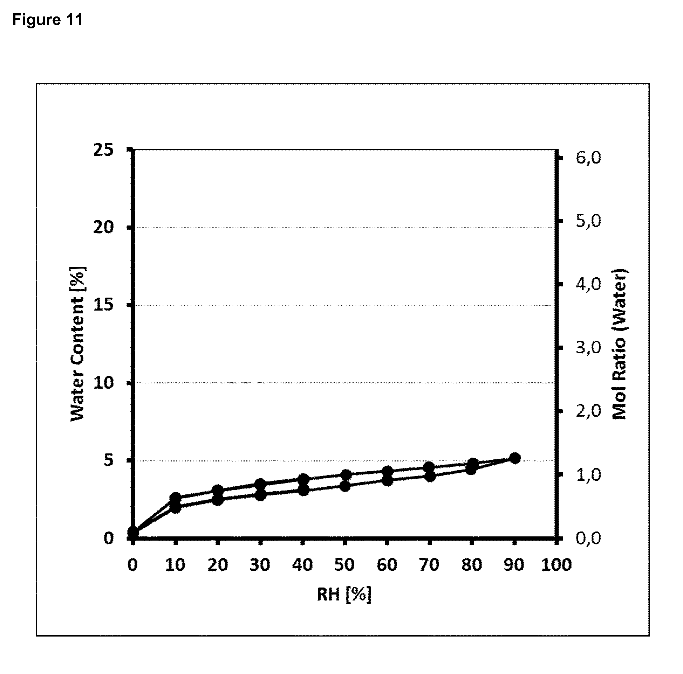

Representative gravimetric moisture sorption/desorption isotherms of form Hy1B are displayed in figure 11 herein. Form Hy1 B, for example, shows a water content of about 2.8 to 3.5% corresponding to 0.7 to 0.9 mol of water per mol of dolutegravir sodium at about 30% RH and a water content of about 4.4 to 4.8% corresponding to 1.1 to 1.2 mol of water per mol of dolutegravir sodium at about 80% RH measured at temperature of 25 ± 0.1 °C. At 90% RH and 25 ± 0.1 °C the sample shows a maximum water content of about 5.1% which corresponds to 1.3 mol of water per mol of dolutegravir sodium. According to GMSD experiments form Hy1 B is a monohydrate.

Hence, alternatively or additionally, form Hy1 B can be characterized as being a monohydrate of dolutegravir sodium.

Alternatively or additionally, form Hy1 B can be characterized by comprising 0.7 to 1.3 mol of water per mol of dolutegravir sodium when measured at 30 to 90% RH and temperature of 25 ± 0.1 °C.

In a still further aspect the present invention relates to a process for the preparation of form Hy1 B of dolutegravir sodium comprising subjecting dolutegravir sodium form S_{EtOH/H2O} of the present invention to an atmosphere having a relative humidity of about at least 75 ± 5% and a temperature of about at least 40 °C.

The exact duration of the transformation from form S_{EtOH/H2O} to form Hy1 B may vary and mainly depends on parameters like sample amount, thickness of the powder layer, particle size, and specific surface area. Usually, the transformation is complete in about 12 to 168 hours, preferably in about 12 to 120 hours, more preferably in about 12 to 72 hours and most preferably the transformation is complete in about 12 to 24 hours.

In a still further aspect the present invention relates to a novel crystalline form of dolutegravir sodium, above and hereinafter also designated form S_{EtOH/H2O}.

Form S_{EtOH/H2O} is a monosodium salt of dolutegravir. Its molar ratio of dolutegravir and sodium typically and preferably lies in the range of about 1.0 : 0.9 to 1.1, preferably of about 1.0 : 1.0.

Form S_{EtOH/H2O} can be characterized by a PXRD comprising at least five peaks at 2-Theta angles selected from 6.5 ± 0.2°, 7.0 ± 0.2°, 10.6 ± 0.2°, 11.0 ± 0.2°, 13.3 ± 0.2°, 16.0 ± 0.2°, 18.0 ± 0.2°, 19.1 ± 0.2°, 20.3 ± 0.2°, 20.8 ± 0.2° and/or 22.6 ± 0.2°.

Preferably, form S_{EtOH/H2O} is characterized by a PXRD comprising characteristic peaks at 2-Theta angles of 6.5 ± 0.2°, 7.0 ± 0.2°, 13.3 ± 0.2°, 16.0 ± 0.2° and 22.6 ± 0.2°. Form S_{EtOH/H2O} may be further characterized by a PXRD further comprising one or more additional characteristic peaks at 2-Theta angles of 10.6 ± 0.2°, 11.0 ± 0.2°, 18.0 ± 0.2°, 19.1 ± 0.2°, 20.3 ± 0.2° and/or 20.8 ± 0.2°. Preferably, form S_{EtOH/H2O} may be further characterized by a PXRD further comprising one or more additional characteristic peaks at 2-Theta angles of 10.6 ± 0.2°, 11.0 ± 0.2°, 18.0 ± 0.2°, 19.1 ± 0.2° and/or 20.3 ± 0.2°.

More preferably, form S_{EtOH/H2O} is characterized by a PXRD comprising characteristic peaks at 2-Theta angles of 6.5 ± 0.2°, 7.0 ± 0.2°, 10.6 ± 0.2°, 11.0 ± 0.2°, 13.3 ± 0.2°, 16.0 ± 0.2°, 18.0 ± 0.2°, 19.1 ± 0.2°, 20.3 ± 0.2° and 22.6 ± 0.2°.

Even more preferably, form S_{EtOH/H2O} is characterized by a PXRD comprising characteristic peaks at 2-Theta angles of 6.5 ± 0.2°, 7.0 ± 0.2°, 10.6 ± 0.2°, 11.0 ± 0.2°, 13.3 ± 0.2°, 16.0 ± 0.2°, 18.0 ± 0.2°, 19.1 ± 0.2°, 20.3 ± 0.2°, 20.8 ± 0.2° and 22.6 ± 0.2°,

XPRDs of form S_{EtOH/H2O} according to the above alternatives are measured preferably with Cu-Kα radiation, more preferably with Cu-Kα_{1,2} radiation having a wavelength of 0.15419 nm at room temperature.

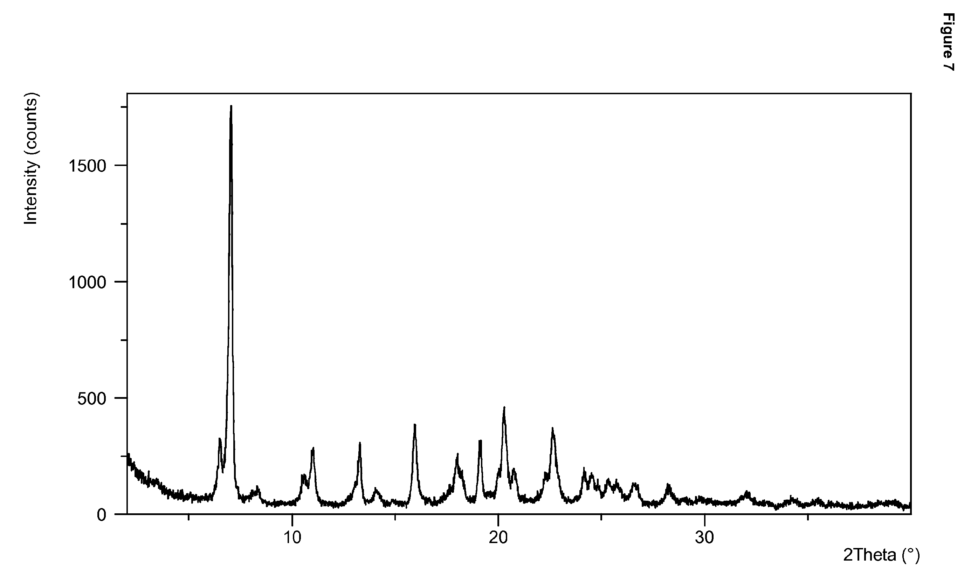

A representative PXRD of form S_{EtOH/H2O} is displayed in figure 7 herein. Additionally, form S_{EtOH/H2O} can be characterized by showing a PXRD essentially the same as displayed in figure 7 when measured at room temperature with Cu-Kα_{1,2} radiation having a wavelength of 0.15419 nm.

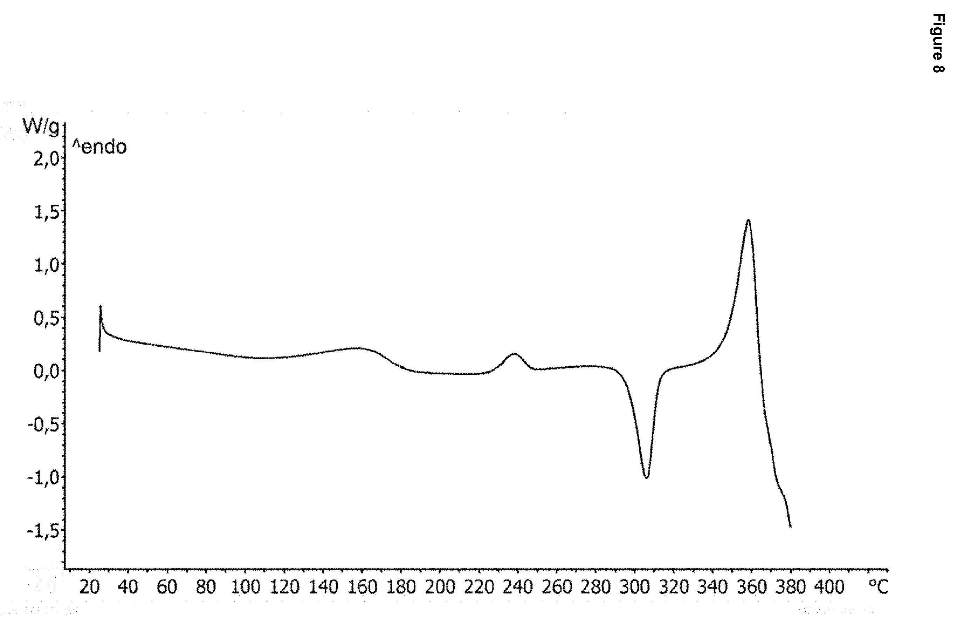

Furthermore, a representative DSC curve of form S_{EtOH/H2O} equilibrated at about 25% RH and RT is displayed in figure 8 herein. The DSC curve of form S_{EtOH/H2O} shows a first endotherm at about 110 to 185 °C with a peak maximum at about 159 °C, which is due to release of ethanol and water respectively. The second endotherm at about 220 to 250 °C with a peak maximum at about 238 °C is caused by melting. The exotherm, which occurs at about 285 to 320 °C and has a peak maximum at about 306 °C indicates that the anhydrous form of WO 2010/068253 A1 crystallizes from the melt. The final endotherm at about 340 to 370 °C with an onset temperature of about 350 °C and a peak maximum at about 358 °C is due to melting and decomposition respectively of the obtained anhydrous form of WO 2010/068253 A1.

Therefore, alternatively or additionally, form S_{EtOH/H2O} can be characterized by a DSC curve comprising an endotherm at about 110 to 185 °C and/or an endotherm at about 220 to 250 °C when measured at a heating rate of 10 °C/min. Form S_{EtOH/H2O} can be further characterized by a DSC curve comprising an additional exotherm at about 285 to 320 °C when measured at a heating rate of 10 °C/min.

Alternatively or additionally, form S_{EtOH/H2O} can be characterized by melting in the range of about 220 to 250 °C when measured with DSC at a heating rate of 10 °C/min.

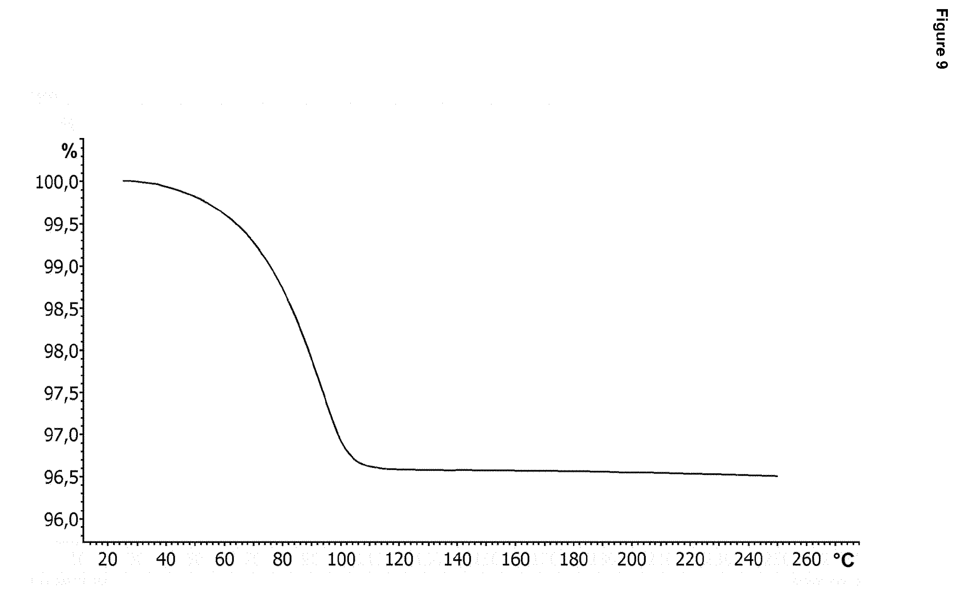

In addition, a representative TGA curve of form S_{EtOH/H2O} equilibrated at about 25% RH and RT is provided in figure 9 herein. The TGA curve of form S_{EtOH/H2O} shows a weight loss of about 3.4% from 25 to 120 °C. Coulometric Karl-Fischer titration of form S_{EtOH/H2O} equilibrated at about 25% RH and room temperature revealed a water content of about 0.9% corresponding to 0.2 mol of water per mol of dolutegravir sodium. Ethanol was detectable by ¹H-NMR. Combining the results from TGA, Karl-Fischer titration and ¹H-NMR it can be concluded that form S_{EtOH/H2O} comprises about 2.5% ethanol corresponding to 0.3 mol of ethanol per mol of dolutegravir sodium and about 0.9% water corresponding to 0.2 mol of water per mol of dolutegravir sodium when measured at about 25% RH and RT.

Hence, alternatively or additionally, form S_{EtOH/H2O} can be characterized as being an ethanolate/ hydrate of dolutegravir sodium.

Alternatively or additionally, form S_{EtOH/H2O} can be characterized by comprising 0.3 mol of ethanol and 0.2 mol of water per mol of dolutegravir sodium when measured at about 25% RH and room temperature.

In a still further aspect the present invention relates to a process for the preparation of form S_{EtOH/H2O} of dolutegravir sodium comprising the steps of:
(i) providing a suspension or a solution of dolutegravir in a solvent comprising at least 90 volume% ethanol
(ii) reacting dolutegravir with sodium ethoxide at a temperature of not more than 45 °C and
(iii) precipitating dolutegravir sodium.

Dolutegravir, which is used as starting material for form S_{EtOH/H2O} production can be prepared in accordance with the procedures disclosed in examples 1a to 1k or 3a to 3k of WO 2010/068253 A1.

In a first step, a suspension or a solution of dolutegravir in a solvent comprising ethanol is prepared. Preferably, the ethanol concentration of the solvent used for preparing the mixture is about at least 90 volume%, more preferably about at least 96 volume% and most preferably about at least 99 volume%. Moreover, the water content of the solvent preferably is about not more than 10 volume%, more preferably about not more than 4 volume% and most preferably about not more than 1 volume%.

The initial suspension or solution applied comprises about 10 to 100 g dolutegravir per liter solvent, preferably about 10 to 75 g, more preferably about 10 to 50 g and most preferably about 10 to 25 g dolutegravir per liter solvent are employed.

In the next step dolutegravir is reacted with sodium ethoxide by adding sodium ethoxide to the mixture or *vice versa.* Sodium ethoxide may be applied as solid or ethanolic solution, whereat solutions which may be applied in the present process have a concentration of about 1 to 25 weight%, preferably of about 10 to 21 weight%, more preferably of about 15-21 weight% and most preferably of about 21 weight%. The ratio of dolutegravir and sodium ethoxide employed is about 1.0: 0.8 to 2.0, preferably about 1.0: 0.9 to 1.5, more preferably about 1.0: 0.9 to 1.2 and most preferably a 1.0: 1.0 ratio is used.

Essentially, when combining sodium ethoxide and dolutegravir, the reaction temperature is kept at about not more than 45 °C, preferably at about not more than 40 °C, more preferably at about not more than 35 °C and most preferably at about not more than 30 °C. The suspension or the solution is kept at the applied temperature for about 1 to 24 hours, preferably for about 1 to 12 hours, more preferably for about 1 to 6 hours and most preferably for about 1 to 2 hours, preferably under stirring.

Subsequently, at least a portion of the obtained dolutegravir sodium may be collected by any conventional method such as filtration or centrifugation, preferably by filtration.

Finally, the isolated dolutegravir sodium crystals may be dried at a temperature of about not more than 80 °C, preferably of about not more than 60 °C, more preferably of about not more than 50 °C and most preferably the crystals are dried at a temperature of about not more than 40 °C for example at about room temperature. Drying may be performed for about 1 to 72 hours, preferably for about 2 to 48 hours, more preferably for about 4 to 24 hours and most preferably for about 6 to 18 hours. Drying may be performed under vacuum preferably at about not more than 100 mbar, more preferably at about not more than 50 mbar and most preferably at about not more than 30 mbar, for example at about 20 to 30 mbar.

Having form S_{EtOH/H2O} in hands, for the first time allows the preparation of the novel monohydrate Hy1 B of the present invention. Form Hy1 B can be prepared by subjecting the form S_{EtOH/H2O} crystals to elevated humidity and temperature. Therefore, form S_{EtOH/H2O} is a valuable intermediate for the preparation of form Hy1 B of the present invention.

Hence, in an eighth aspect the present invention relates to the use of dolutegravir sodium form S_{EtOH/H2O} for the preparation of dolutegravir sodium form Hy1 B.

WO 2010/068253 A1 discloses a monohydrate of dolutegravir sodium from which the non-stoichiometric hydrate HxA of the present invention can for example be differentiated by comprising PXRD peaks at 12.5 and 20.8 ± 0.2° 2-Theta, whereas the known monohydrate does not show significant peaks in this area. On the other hand the monohydrate Hy1 B of the present invention differs from the monohydrate disclosed in WO 2010/068253 A1 for example by comprising PXRD peaks at 6.3 and 7.4 ± 0.2° 2-Theta, whereas the known monohydrate does not show significant peaks in this area. Instead, the monohydrate of WO 2010/068253 A1 shows for example PXRD peaks at 8.0 and 9.3 ± 0.2° 2-Theta, whereas the monohydrate Hy1 B of the present invention shows no peaks in this area.

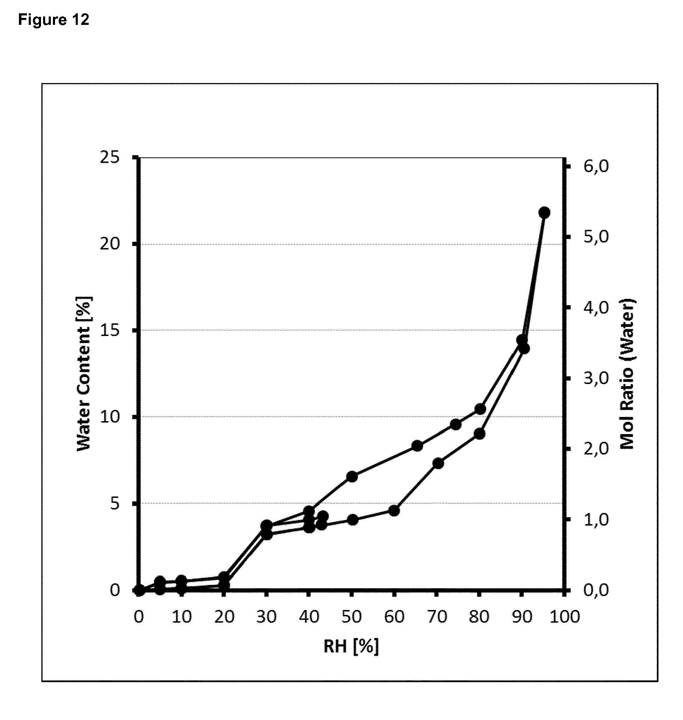

Comparative gravimetric moisture sorption/desorption experiments at 25 ± 0.1 °C were conducted with dolutegravir sodium monohydrate of WO 2010/068253 A1 and the hydrates of the present invention designated form HxA and form Hy1 B. The resulting sorption/ desorption isotherms of the monohydrate of WO 2010/068253 A1 are displayed in figure 12, whereat the isotherms of the non-stoichiometric hydrate HxA and the monohydrate Hy1 B of the present invention are disclosed in figures 10 and 11 respectively.

Below 30% RH dolutegravir sodium monohydrate of WO 2010/068253 A1 shows reversible dehydration to an anhydrous form. On the other hand, above 60% relative humidity the monohydrate excessively takes up water until a water content of about 21.8% is reached at 95% RH, which corresponds to 5.6 mol of water per mol of dolutegravir sodium. Although, this water uptake is reversible when humidity is decreased, the hysteresis between the sorption and desorption isotherms in the area from about 40 to 90% RH indicates significant structural changes. Therefore, it can be concluded that dolutegravir sodium monohydrate of WO 2010/068253 A1 is very hygroscopic and preserves its crystal structure only within a narrow window of 30 to 60% RH.

On the contrary, form HxA of the present invention only shows slight water uptake in the sorption cycle until a water content of 3.9% is reached at 95% RH. The water uptake during the sorption cycle is reversible when humidity is decreased. The absence of a hysteresis between the sorption and desorption isotherms indicates that the crystal structure remains stable during the whole experiment, which was confirmed by moisture dependent PXRD. In summary, dolutegravir sodium form HxA of the present invention is stable and preserves its crystal structure from about 0 to 95% RH and is significantly less hygroscopic than the monohydrate of WO 2010/068253 A1.

Form Hy1 B also shows only slight water uptake in the sorption cycle until a water content of 5.1% is reached at 90% RH. The water uptake is reversible when humidity is decreased. The absence of a significant hysteresis between the sorption and desorption isotherms indicates that the crystal structure remains stable between 10 to 90% RH, which was confirmed by moisture dependent PXRD. Only below 10% RH reversible dehydration to an anhydrous form of dolutegravir sodium can be observed. Therefore, dolutegravir sodium form Hy1 B of the present invention is stable and preserves its crystal structure from 10 to 90% RH and is significantly less hygroscopic than the monohydrate of WO 2010/068253 A1.

The results of the GMSD experiments are again summarized in table 1 below:

**Table 1: Summary of the results from comparative GMSD experiments**

| | **Δm sorption [0-90% RH]** | **Δm sorption [30-90% RH]** | **Remarks** |
|---|---|---|---|
| Form HxA | +3.4% | +2.5% | stable at 0-95% RH no structural changes observed no significant hysterises |
| Form Hy1B | +4.8% | +2.4% | stable at 10-90% RH, dehydration below 10% RH no significant hysterises |
| MH of WO 2010/068253 | +14.0% | +10.7% | stable at 30-60% RH, dehydration below 30% RH, hysterises at 40-95% RH |

Hence, scope of the present invention was the provision of novel hydrates of dolutegravir sodium which absorb only low amounts of water when subjected to moisture and preserve their crystal structure at wet and dry conditions. Due to their superior properties, the hydrates of the present invention can be easily formulated as well as packaged and stored in a controlled way.

Therefore, in a further aspect the present invention relates to the use of dolutegravir sodium form HxA and/or form Hy1 B for the preparation of pharmaceutical compositions.

In addition, the present invention relates to pharmaceutical compositions comprising an effective amount of dolutegravir sodium form HxA and/or form Hy1 B and at least one pharmaceutically acceptable excipient.

Finally, the present invention relates to pharmaceutical compositions comprising an effective amount of dolutegravir sodium form HxA and/or form Hy1 B for use in the treatment of human immunodefiency virus type 1 (HIV-1) infection.

The following non-limiting examples are illustrative for the disclosure.

### EXAMPLES

PXRDs were obtained with an X'Pert PRO diffractometer (PANalytical, Almelo, The Netherlands) equipped with a theta/theta coupled goniometer in transmission geometry, programmable XYZ stage with well plate holder, Cu-Kα_{1,2} radiation source (wavelength 0.15419 nm) with a focussing mirror, a 0.5° divergence slit, a 0.02° soller slit collimator and a 0.5° anti-scattering slit on the incident beam side, a 2 mm anti-scattering slit, a 0.02° soller slit collimator, a Ni-filter and a solid state PIXcel detector on the diffracted beam side. The diffractogram was recorded at room temperature at a tube voltage of 40 kV, tube current of 40 mA, applying a stepsize of 0.013° 2-Theta with 40 sec per step in the angular range of 2° to 40° 2-Theta. A typical precision of the 2-Theta values is in the range of ± 0.2° 2-Theta. Thus, the diffraction peak of Form HxA that appears for example at 9.4° 2-Theta can appear between 9.2 and 9.6° 2-Theta on most X-ray diffractometers under standard conditions.

DSC was performed on a Mettler Polymer DSC R instrument. The samples were heated in 40 µL aluminum pans with pierced aluminum lids from 25 to 380°C at a rate of 10 °C/ min. Nitrogen (purge rate 50 mL/min) was used as purge gas.

TGA was performed on a Mettler TGA/ DSC 1 instrument. The samples were heated in 100 µL aluminum pans closed with aluminum lids. The lids were automatically pierced at the beginning of the measurements. The samples were heated from 25 to 250 °C at a rate of 10 °C/ min. Nitrogen (purge rate 50 mL/ min) was used as purge gas.

Coulometric Karl-Fischer titrations were performed on a Metrohm 831 KF Coulometer.

The water values at varying relative humidities were obtained by recording moisture sorption/ desorption isotherms with a SPS-11 moisture sorption analyzer (MD Messtechnik, Ulm, D). The samples were weighed into Teflon sample holders. The measurement cycles of the monohydrate of WO 2010/068253 A1 and the non-stoichiometric hydrate HxA of the present invention were started at 43% RH, decreased to 40% RH, further decreased in 10% steps to 10% RH, decreased in 5% steps to 0% RH, increased in 5% steps to 10% RH, further increased in 10% steps to 90% RH and subsequently increased to 95% RH, decreased again to 90% RH, decreased in 10% steps to 10% RH, further decreased in 5% steps to 0% RH, again increased in 5% steps to 10%, subsequently increased in 10% steps to 40% RH and finally increased to 43% RH. The measurement cycle of the monohydrate Hy1 B of the present invention was started at 40% RH decreased in 10% steps to 0% RH, increased in 10% steps to 90% RH, decreased in 10% steps to 0% RH, and finally increased in 10% steps to 40% RH. The equilibrium condition for each step was set to a mass constancy of ± 0.005% over 60 min. The temperature was 25 ± 0.1 °C. The water content of the samples was determined after the moisture sorption/ desorption experiments with a TGA 7 system (Perkin Elmer, Norwalk, Ct., USA) using the Pyris 2.0 software. The samples were weighed into Al pans (50 µL). Dry nitrogen was used as purge gas (purge rate: 20 mL/min). The samples were heated from 25 to 200 °C using a heating rate of 10 °C/min.
¹H-NMR spectra were recorded at 500 MHz on a Bruker Avance 500 instrument. Deuterated dimethyl sulfoxide (99.9%D) with tetramethylsilane as internal standard was used as solvent.

### Example 1: Preparation of dolutegravir sodium form HxA

A suspension of dolutegravir (6.006 g, 14.3 mmol, for example prepared in accordance with the procedures disclosed in examples 1 a to 1k of WO 2010/068253 A1) in 78 mL methanol was cooled to 5 ± 1 °C. A refrigerated solution of sodium methoxide in methanol (25 weight%, 3.282 mL, 14.4 mmol) was dropwise added under stirring and the obtained suspension was further stirred at 5 ± 1 °C for 2 h. Thereafter, the temperature of the suspension was increased to 25 ± 1 °C in 1 h and stirred at the same temperature for additional 21 h. Finally, the crystals were collected by filtration and dried at RT under vacuum (20-30 mbar) for 21.5 h to obtain 5.843 g of dolutegravir sodium form HxA.
Yield: 92% of theory;

The PXRD of the obtained sample is displayed in figure 1 and the corresponding peak list is provided in table 2 below:

**Table 2: PXRD peak list of dolutegravir sodium Form HxA obtained according to example 1**

| **Position [± 0.2 °2Th.]** | **Relative Intensity [%]** | **Position [± 0.2 °2Th.]** | **Relative Intensity [%]** |
|---|---|---|---|
| 7.9 | 42 | 19.8 | 7 |
| 9.4 | 100 | 20.0 | 8 |
| 11.4 | 15 | 20.8 | 17 |
| 11.6 | 16 | 22.9 | 17 |
| 12.5 | 12 | 23.3 | 8 |
| 12.8 | 6 | 24.4 | 19 |
| 13.6 | 13 | 25.2 | 7 |
| 15.2 | 9 | 26.0 | 12 |
| 15.9 | 37 | 27.1 | 10 |
| 18.4 | 6 | 28.4 | 5 |
| 19.1 | 8 | 29.4 | 5 |

### Example 2: Preparation of dolutegravir sodium form HxA

A suspension of dolutegravir (1.007 g, 2.4 mmol, for example prepared in accordance with the procedures disclosed in examples 1 a to 1 k of WO 2010/068253 A1) in 13 mL methanol was adjusted to a temperature of 25 ± 1 °C. Solid sodium methoxide (assay: 95%, 0.136 g, 2.4 mmol) was added and the obtained suspension was further stirred at 25 ± 1 °C for 2 hours. Finally, the crystals were collected by filtration and dried at RT under vacuum (20-30 mbar) for 19 h to obtain 0.965 g of dolutegravir sodium form HxA.
Yield: 91% of theory; Karl-Fischer titration: 0.9%; TGA: -0.9% (25-120 °C, 10 °C/min)

### Example 3: Preparation of dolutegravir sodium form HxA at different reaction temperatures

A suspension of dolutegravir (1.0 g, 2.4 mmol, for example prepared in accordance with the procedures disclosed in examples 1 a to 1 k of WO 2010/068253 A1) in 13 mL methanol was adjusted to a temperature according to table 3. Solid sodium methoxide (assay: 95%, 0.136 g, 2.4 mmol) was added and the obtained suspension was further stirred at the applied temperature for 2 hours. Finally, the crystals were collected by filtration and dried at RT under vacuum (20-30 mbar) for 19 h. The resulting materials were investigated by PXRD and the results are summarized in table 3.

**Table 3: Crystalline forms obtained from reactions performed at different temperatures**

| **Example** | **Temperature** | **Assignment according to PXRD** |
|---|---|---|
| 3a | 10 | HxA |
| 3b | 15 | HxA |
| 3c | 20 | HxA |
| 3d | 25 | HxA |
| 3e | 30 | HxA |
| 3f | 40 | HxA + anhydrous form of WO 2010/068253 A1 |
| 3g | 50 | anhydrous form of WO 2010/068253 A1 + HxA |

### Example 4: Preparation of dolutegravir sodium form HxA using different concentrations

A suspension of dolutegravir (1.0 g, 2.4 mmol respectively 0.5 g, 1.2 mmol, for example prepared in accordance with the procedures disclosed in examples 1a to 1k of WO 2010/068253 A1) in an amount of methanol according to table 4 was adjusted to a temperature of 25 ± 1 °C. Solid sodium methoxide (assay: 95%, 0.136 g, 2.4 mmol respectively 0.068 g, 1.2 mmol) was added and the obtained suspension was further stirred at 25 ± 1 °C for 2 hours. Finally, the crystals were collected by filtration and dried at RT under vacuum (20-30 mbar) for 19 h. The resulting materials were investigated by PXRD and the results are summarized in table 4.

**Table 4: Crystalline forms obtained from reactions performed with different concentrations**

| **Example** | **m_{dolutegravir}** | **V_{MeOH}** | **Conc.** | **Assignment according to PXRD** |
|---|---|---|---|---|
| 4a | 0.5 g | 50 mL | 10 g/L | anhydrous form of WO 2010/068253 A1 + HxA |
| 4b | 0.5 g | 17 mL | 30 g/L | anhydrous form of WO 2010/068253 A1 + HxA |
| 4c | 0.5 g | 10 mL | 50 g/L | anhydrous form of WO 2010/068253 A1 + HxA |
| 4d | 1.0 g | 13 mL | 75 g/L | HxA |
| 4e | 1.0 g | 10 mL | 100 g/L | HxA |

### Example 5: Preparation of dolutegravir sodium form Hy1B

Dolutegravir sodium form S_{EtOH/H2O} (prepared according to example 6 herein) was subjected for 5 days to an atmosphere having a temperature of about 40 °C and a relative humidity of about 75 % to obtain form Hy1 B quantitatively.
Karl-Fischer titration: 3.3%; TGA: -3.3% (25-140 °C; 10 °C/min)

The PXRD of the obtained sample is displayed in figure 4 and the corresponding peak list is provided in table 5 below:

**Table 5: PXRD peak list of dolutegravir sodium Form Hy1B obtained according to example 4**

| **Position [± 0.2 °2Th.]** | **Relative Intensity [%]** | **Position [± 0.2 °2Th.]** | **Relative Intensity [%]** |
|---|---|---|---|
| 6.3 | 22 | 19.7 | 15 |
| 7.4 | 100 | 20.5 | 17 |
| 10.9 | 18 | 21.0 | 19 |
| 11.1 | 18 | 22.1 | 13 |
| 12.7 | 19 | 23.0 | 29 |
| 16.2 | 30 | 24.0 | 14 |
| 18.6 | 24 | 24.9 | 10 |
| 18.8 | 28 | 26.5 | 13 |

### Example 6: Preparation of dolutegravir sodium form S_{EtOH/H2O}

A suspension of dolutegravir (2.004 g, 4.8 mmol, for example prepared in accordance with the procedures disclosed in examples 1a to 1k of WO 2010/068253 A1) in 80 mL ethanol was adjusted to a temperature of 25 ± 1 °C. Solid sodium ethoxide (assay: 95%, 0.516 g, 7.2 mmol) was added and the obtained suspension was further stirred at 25 ± 1 °C for 2 h. Finally, the crystals were collected by filtration and dried at RT under vacuum (20-30 mbar) for 42 h to obtain 2.105 g of dolutegravir sodium form S_{EtOH/H2O}.
Yield: 96% of theory; Karl-Fischer titration: 0.9%; TGA: -3.4% (25-120 °C, 10 °C/min)

The PXRD of the obtained sample is displayed in figure 7 and the corresponding peak list is provided in table 6.

**Table 6: PXRD peak list of dolutegravir sodium Form S_{EtOH/}_{H2O} obtained according to example 5**

| **Position [± 0.2 °2Th.]** | **Relative Intensity [%]** | **Position [± 0.2 °2Th.]** | **Relative Intensity [%]** |
|---|---|---|---|
| 6.5 | 16 | 18.0 | 12 |
| 7.0 | 100 | 19.1 | 15 |
| 10.6 | 7 | 20.3 | 24 |
| 11.0 | 14 | 20.8 | 8 |
| 13.3 | 16 | 22.6 | 19 |
| 16.0 | 20 | | |

### Example 7: Preparation of dolutegravir sodium form S_{EtOH/H2O} at different reaction temperatures

A suspension of dolutegravir (0.5 g, 1.2 mmol for example prepared in accordance with the procedures disclosed in examples 1 a to 1k of WO 2010/068253 A1) in 50 mL ethanol was adjusted to a temperature according to table 7. Solid sodium ethoxide (assay: 95%, 0.085 g, 1.2 mmol) was added and the obtained suspension was further stirred at the applied temperature for 2 hours. Finally, the crystals were collected by filtration and dried at RT under vacuum (20-30 mbar) for 20 h. The resulting materials were investigated by PXRD and the results are summarized in table 7.

**Table 7: Crystalline forms obtained from reactions performed at different temperatures**

| **Example** | **Temperature** | **Assignment according to PXRD** |
|---|---|---|
| a | 5 | HxA |
| b | 15 | HxA |
| c | 25 | HxA |
| d | 35 | HxA |
| e | 45 | HxA |
| f | 60 | anhydrous form of WO 2010/068253 A1 + HxA |

### Reference Example 1 - Preparation of dolutegravir sodium monohydrate of WO 2010/068253 A1

A mixture of dolutegravir (1.998 g, 4.8 mmol, for example prepared in accordance with the procedures disclosed in examples 1a to 1k of WO 2010/068253 A1) in 40 mL THF/water (8: 2 = V: V) was adjusted to a temperature of 30 °C. After the addition of 2.4 mL 2N NaOH the mixture was stirred at 25 ± 1°C for 2 h. The obtained crystals were collected by filtration, washed with 10 mL THF/ water (8: 2 = V: V) and dried at 85 °C under vacuum (20-30 mbar) for 18 h to obtain 1.845 g of dolutegravir sodium in form of the monohydrate disclosed in WO 2010/068253 A1.

## Claims

1. A crystalline form of dolutegravir sodium of formula (I) 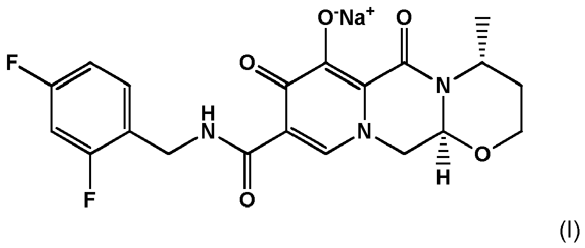 **characterized by** a powder X-ray diffractogram comprising peaks at 2-Theta angles of 7.9 ± 0.2°, 9.4 ± 0.2°, 12.5 ± 0.2°, 15.9 ± 0.2° and 20.8 ± 0.2°.

2. The crystalline form according to claim 1 comprising 0.2 to 0.7 mol of water per mol dolutegravir sodium of formula (I) when measured at a relative humidity from 30 to 70% and a temperature of 25 ± 0.1 °C.

3. A process for the preparation of the crystalline form according any one of the claims 1 to 2 comprising:
(i) providing a suspension or a solution of dolutegravir of formula (II) 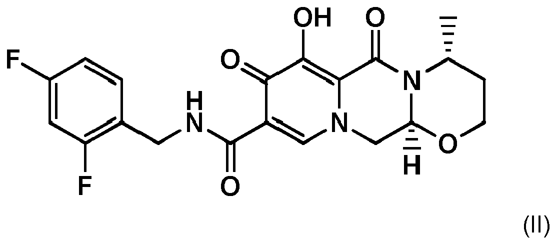 in a solvent, preferably comprising at least 90 volume% methanol, wherein the suspension or the solution comprises more than 50 g, preferably at least 75 g dolutegravir of formula (II) per liter solvent,
(ii) reacting dolutegravir of formula (II) with sodium methoxide at a temperature of not more than 30 °C and
(iii) precipitating dolutegravir sodium.

4. The process according to claim 3, further comprising
(iv) separating at least a portion of the crystalline form of dolutegravir sodium according to any one of the claims 1 to 2 from its mother liquor.

5. A crystalline form of dolutegravir sodium of formula (I) 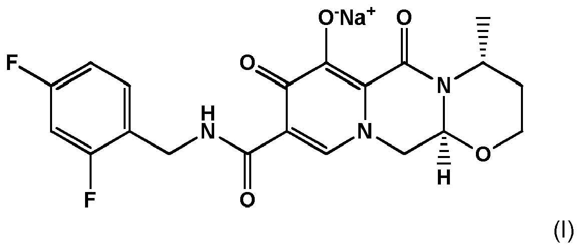 **characterized by** a powder X-ray diffractogram comprising peaksand/or at 2-Theta angles of 6.3 ± 0.2°, 7.4 ± 0.2°, 11.1 ± 0.2°, 12.7 ± 0.2° and 16.2 ± 0.2°.

6. The crystalline form according to claim 5 comprising 0.7 to 1.3 mol of water per mol dolutegravir sodium of formula (I) when measured at a relative humidity from 30 to 90% and a temperature of 25 ± 0.1 °C.

7. A crystalline form of dolutegravir sodium of formula (I) 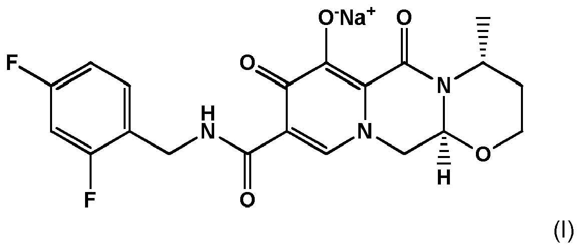 **characterized by** a powder X-ray diffractogram comprising peaksand/or at 2-Theta angles of 6.5 ± 0.2°, 7.0 ± 0.2°, 13.3 ± 0.2°, 16.0 ± 0.2° and 22.6 ± 0.2°.

8. The crystalline form according to claim 7 comprising 0.3 mol of ethanol and 0.2 mol of water per mol dolutegravir sodium of formula (I) when measured at a relative humidity of 25% and a temperature from 15 to 25 °C.

9. A process for the preparation of the crystalline form according any one of the claims 5 to 6 comprising subjecting the crystalline form according any one of the claims 7 to 8 to an atmosphere having a relative humidity of at least 75 ± 5% and a temperature of at least 40 °C.

10. A process for the preparation of the crystalline form according to any one of the claims 7 to 8 comprising the steps of:
(i) providing a suspension or solution of dolutegravir of formula (II) 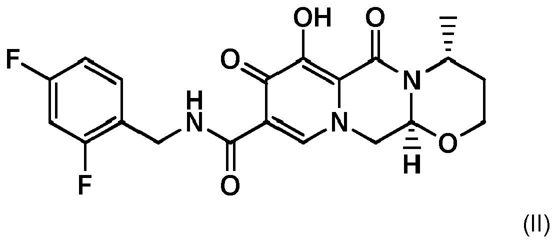 in a solvent preferably comprising at least 90 volume% ethanol, wherein the suspension or the solution preferably comprises 10 g to 100 g dolutegravir of formula (II) per liter solvent,
(ii) reacting dolutegravir of formula (II) with sodium ethoxide at a temperature of not more than 45 °C and
(iii) precipitating dolutegravir sodium.

11. The process according to claim 10, further comprising
(iv) separating at least a portion of the crystalline form of dolutegravir sodium according to any one of the claims 7 to 8 from its mother liquor.

12. Use of the crystalline form according to any one of claims 1 or 2 and/or 5 or 6 or mixtures thereof for the preparation of a pharmaceutical composition.

13. A pharmaceutical composition comprising an effective amount of the crystalline form according to any one of claims 1 or 2 and/or 5 or 6 or mixtures thereof and at least one pharmaceutically acceptable excipient.

14. The pharmaceutical composition according to claim 13 for use in a method for treating immunodefiency virus type 1 (HIV-1) infection in human.

15. Use of the crystalline form according to any one of the claims 7 to 8 for the preparation of the crystalline form according to any one of the claims 5 to 6.
